Europäisches Patentamt

European Patent Office

Office européen des brevets

(11) Publication number: **0 366 869**
**A2**

(12) **EUROPEAN PATENT APPLICATION**

(21) Application number: **89112992.6**

(22) Date of filing: **14.07.89**

(51) Int. Cl.5: **A61K 37/56 , A61K 35/54 ,**
**A61K 33/00 , A61K 39/39 ,**
**A01N 59/00 , A01N 63/02 ,**
**A23C 3/00 , A23L 3/00 ,**
**A61K 7/40**

(30) Priority: **17.10.88 US 258606**

(43) Date of publication of application:
**09.05.90 Bulletin 90/19**

(84) Designated Contracting States:
**AT BE CH DE ES FR GB GR IT LI LU NL SE**

(71) Applicant: **LYCON AG**
**Neugasse 29**
**CH-6300 Zug(CH)**

(72) Inventor: **Sapse, Alfred T., Dr.**
**1444 Biscayne Boulevard Suite 220**
**Miami Florida 33132(US)**

(74) Representative: **von Hellfeld, Axel, Dr.**
**Dipl.-Phys., et al**
**WUESTHOFF & WUESTHOFF**
**Schweigerstrasse 2**
**D-8000 München 90(DE) .**

(54) **Bacteriostatic, bactericidal and antifungal composition and methods of use thereof.**

(57) A bactericidal, bacteriostatic and antifungal composition comprising lysozyme, a mineral component and an immunomodulating agent and use of said composition for reducing or inhibiting the bacteriological of fungal activity in various substrates.

EP 0 366 869 A2

# BACTERIOSTATIC, BACTERICIDAL AND ANTIFUNGAL COMPOSITION AND METHODS OF USE THEREOF

## Technical Field

The present invention relates to a composition having bactericidal, bacteriostatic and antifungal activity. The composition comprises lysozyme, a mineral component, and an immunomodulating agent, preferably a fatty acid, working in a synergistic way.

They can be used as individual components using the right dosage, pH and temperature, or in salt(s) form, which would reverse to original compounds when in solution, or irreversible covalent binding complexes.

This invention also encompasses a method for use of such composition for the reduction or inhibition of bacteriological activity in a substrate, a method for the extension of the shelf life of fresh foods and a method for extending the shelf life of milk and milk products and inducing the curding thereof.

Lysozyme is an antibiotic enzyme found in humans, animals and plants. In humans, it plays an important role in the immune system, acting against bacterial and viral infections. It is generally found in tears, nasal mucus, milk, saliva, blood serum, in a great number of tissues and secretions of different animals, vertebrates and invertebrates, in egg white, in some molds and in the latex of different plants, and was first discovered in human tears in 1922 by Alexander Fleming and first isolated and characterized in 1967 by Alfred Sapse, B. Bonavida and E.E. Sercarz at the University of California at Los Angeles (Bonavida et al., Human Tear Lysozyme: I. Purification, Physicochemical and Immunochemical Characterization, J. Lab. Clin. Med. 70:951, 1967).

Lysozyme alone has been found to have very limited bactericidal effects and is synergistically aided in vivo by certain proteins. Included among these is transferrin (a carrier of iron) and ceruloplasmin (a carrier of copper); the immunomodulator proteins immunoglobulin A (IgA) and immunoglobulin G (IgG); and, in tears, by tear prealbumin.

Hen egg white lysozyme is a lysozyme isolated from the whites of hen eggs and is readily available. As is the case with tear lysozyme, hen egg white lysozyme has a very low bactericidal and bacteriostatic activity in and of itself. What is needed, therefore, is a composition which causes the bactericidal and bacteriostatic activity of lysozyme in vitro to approximate that of human lysozyme in vivo.

## Background Art

The antibacterial activity of lysozyme has been recognized both in terms of lysozyme alone, as in Japanese Patent No. 72-46336, as well as in combination with other ingredients such as glycine (in Japanese Patent No. 73-16613) and hydrogen peroxide with certain acids or acid salts (in German Patent No. 2,126,204). Unfortunately, though, the synergistic effect of the combination of lysozyme with an immunomodulator and a mineral at an acidic pH on the antibacterial activity of lysozyme has never been recognized or disclosed.

## Disclosure of Invention

The present invention relates to a bactericidal, bacteriostatic and antifungal composition comprising lysozyme, most preferably hen egg-white lysozyme, and additionally containing a mineral component, most preferably on zinc and/or iodine basis, and an immunomodulating agent, most preferably a fatty acid.

The lysozyme can be of animal origin (hen egg-white, or turkey egg-white), vegetable origin (yam/sweet potato), human (serum, placenta), or bioengineered.

The fatty acids which are preferably used, can be selected from the saturated, such as acetic, butyric, caprylic, (caproic acid is of little use because of its poignant smell), lauric, myristic, palmitic, stearic, and arachidic) or unsaturated, such as oleic, elaidic, cis- and trans-vaccenic, linoleic, linolenic and arachidonic, fatty acids.

Sometimes they can be used in hydroxy from or as methyl or cholesteryl esters. All and every fatty acid component, mentioned above, has, by itself, a certain mycobactericidal effect, that as it will be shown in detail, can be greatly enhanced when used synergically with lysozyme and certain minerals, according with our technique, detailed further down.

The minerals preferably chosen, but not limited to, comprise either iodine or zinc. While each of them has, under certain conditions, antibacterical or antifungal activity, their action is greatly increased when used synergistically with lysozyme and the fatty acid(s) mentioned above.

The range of lysozyme preferably varies between 20 - 200 mg/ml or 20 - 200 ppm., a milliliter containing the amount of composition needed for one kg or liter of food to be preserved or substrate to be treated.

Fatty Acids: 50 - 500 mg/ml or 50 - 500 ppm.

Zinc: 2 - 25 mg/ml or 2 - 25 ppm.

Iodine: 125 - 500 $\mu$g/ml or 0.125 to 0.5 ppm.

While preparations are, in general, effective when combined within this range, the optimum dosage range is, under certain conditions, very narrow; and when not using the exact amounts of each ingredient, and optimum pH and temperature, these ingredients tend to get out of the solution or precipitate.

The pH range of activity is between 3.5 - 12.0.

The temperature range is from 20°C to 90°C.

The product is thermostable for up to sixty (60) hours, and 80°C without any loss of activity.

The composition of the invention has antibacterial activity equal with 50 units of cephalexin, or 500 units of gentamicin, or higher than 500 units of the antifungal nystatin.

## Antibacterial Activity

The formulations were tested against the following Gram positive and Gram negative bacteria:

Salmonella typhimurium, Salmonella enteriditis, Listeria monocytogenes. Clostridium botulinum, Closteridium perfringens, E. coli, Staphaureus, Campylobacter jejuni, Vibrio parahemolyticus, Pseudomonas aeruginosa, Pseudomonas fluorescens, Aspergillus niger.

The composition has been tested against the bacteria mentioned above in the following foods:

- in liquid Form - in egg preparations including mayonnaise meats, hen, turkey, ham as canned and non-canned foods, soups, salami, sausages, smoked products; also in
- fish products in ground form, including canned food, in cheeses, milk products, soft drinks including natural juices;
- in spray Form - on carcasses, meats, fish, lobster, shrimp and frog legs, also in spices heavily contaminated with salmonella, including black pepper and cinnamon.
- as a preservative in animal feeds - used either in drinking water, dry food (chow), in gelatin and solid cookies, in preveting contamination and transuterine contamination with Salmonella and others in a poultry population.

## Antifungal Activity

Effectiveness was shown against Candida albicans, geotrichum (fungus afflicting dairy products), Brewer's yeast, when used directly against them in laboratory tests, or in foods experimentally contaminated, such as yogurt, soft drinks, including colas and natural juices.

In the wet corn milling, the composition according to the invention alone can partially replace the sulfites, whereas, combined with an enzymatic, wet-milling (cellulase) product, the composition is capable of completely replacing sulfites.

Partial replacement of sulfites can occur in the wine industry, mustard, spraying of fresh and dry fruits and vegetable.

Partial replacement of nitrites and nitrates can occur in the sausage and ham industry, when used alone, or total replacement in combination with natural, red colors and ascorbic acid, ascorbate or erythorbic acid.

There are a number of non-food uses of the composition of the invention, as follows:

In Mouthwashes. The composition is highly effective in destroying mouth infection-inducing bacteria such as Streptococcus salivarius, Bacteroides vulgatus, and Candida albicans and others, when used directly against, or when mixed with commercial mouthwashes.

In Denture Cleaners. A few drops of the composition induces both antibacterial and anticandida capabilities, to commercial preparations previously devoid of this action.

In Contact Lens Solutions. A few drops added to the unpreserved commercial lens solutions confers antibacterial activity against Pseudomonas cepacia and Serratia marcenses, equal to or stronger than the commonly used mercury salts (thiomersal), benzalkonium chloride, etc.

In Cosmetics. In ointment form, creams, shampoos, talcum powder, deodorants, nail polishes, (eliminates potential fungus infection), mascara, eyelid products.

In Feminine Hygiene. Vaginal douches, and in preventing and suppressing bacterial and fungal infections associated with the use of panties.

In Diapers. In preventing contamination with bacteria and fungus.

With Bed Sores, especially in a bedridden geriatric population.

Salts were prepared from lysozyme and the fatty acid component, such as :

Lysozyme acetate, butyrate, caprylate, laureate, linolinate and the other individual fatty acids mentioned previously also. Salts were produced from mixing the mineral with a fatty acid resulting in zink acetate, butyrate, caprylate, laureate, etc., or, conversely, iodine acetate, butyrate, caprylate laureate, linolenate, etc., and finally mixing the two types of salts at pH 3.5. to 12.0 at 20 - 50° C, preferably at 25° C.

Compositions with covalently bound lysozyme - fatty acid complexes may be obtained and use.

These complexes are prepared using lysozyme and a fatty acid in the presence of Ethyl-3-(dimethylaminopropyl)carbodiimide on a mole per mole basis; also similarly between the fatty acid and the mineral.

As noted above, the present invention further relates to a composition comprising lysozyme, most preferably hen egg-white lysozyme, a mineral component, and immunomodulating agent and is at a pH of composition having bactericidal and bacteriostatic activity.

This invention als encompasses a method of use of such composition of the reduction or inhibition of bacteriological activity in a substrate, a method for the extension of the shelf life of fresh foods and a method for inducing curding in milk and milk products. As used therein. the terms "bacteria", "bacterial", etc., shall also be understood to include "fungus", "fungal", etc.

Lysozyme (N-acetylmuramide glycanohydrolase; globulin $G_1$) is a mucolytic enzyme having antibiotic properties. Its molecular weight is about 14,500 and its structure consists of a single polypeptide chain of 129 amino acid subunits of 20 different types, crosslinked by four disulfide bridges. Although not intending to be bound by any particular theory, it is thought that lysozyme exhibits its antibacterial activity (i.e., its bactericidal and bacteriostatic activity) by dissolving the bacterial cell wall mucopolysaccharides by hydrolyzing the beta-(1 through 4) linkages between the N-acetyl-D-muramic acid and 2-acetylamino-2-deoxy-D-glucose residues.

In the description, concentrations given in ppm relate to final compositions resulting from treatment of a substrate with more concentrated compositions of the invention, whereas concentrations given in mg/ml or μg/ml relate to concentrate compositions for treating a substrate on a ml per kg or 1 basis.

An antibacterial composition which comprises lysozyme advantageously comprises about 0.5 parts per million (ppm) to about 500 ppm of the lysozyme. Most preferably, such a composition comprises about 5 ppm to about 40 ppm of the lysozyme. The preferred lysozyme is hen egg white lysozyme due to its commercial availability, although any isolated lysozyme can be utilized.

The composition of this invention preferably also contains a mineral, most preferably zinc. It has surprisingly been found that zinc contributes synergistically to the activity of lysozyme, since it had been commonly thought that zinc, when added in certain concentrations to oral supplements or alimentation fluids, may contribute directly or indirectly to microbial growth or worsen infections.

Other minerals in addition to zinc which have found to be effective include, without limitation, metals such as germanium, beryllium, chromium, tungsten, selenium, iron, copper, manganese, superoxide dismutase, magnesium, strontium and iodine/kelp. Mixtures of the above minerals may also be utilized. As noted, zinc is most preferred and can also be provided as zinc sulfate, zinc oxide, zinc gluconate and other salts and compounds of zinc.

The mineral component is preferably present in the composition of the present invention in an amount of about 0.5 ppm to about 50 ppm, most preferably about 1 ppm to about 4 ppm, for effectiveness, although there has not been found to be any criticality in these proportions and it will be recognized that slightly more or slightly less will not significantly impact on the efficacy of the inventive composition.

Also present in the composition of this invention is an immunomodulator, which can provide the synergistic benefits provided by IgA and IgG. A suitable immunomodulator is an acid, most particularly ascorbic acid. Other suitable acids which may be included as the immunomodulator are pantothenic acid, folic acid, benzoic acid, tartaric acid, para-amino benzoic acid, linoleic acid, gammalinoleic acid, and other organic and inorganic acids. Of particular importance is acetylsalicylic acid and its derivatives. Ascorbic acid can also be replaced in the inventive composition by amino acids such as glutamic acid, alanine, glycine, methionine, phenylalanine, tryptophan, etc.; and fatty acids such as caprylic acid, lauric acid and others. As was the case with the mineral component, the immunomodulator can also comprise a mixture of the above-mentioned acids.

4

The immunomodulator is advantageously present in an amount of about 6 to about 300 ppm. Most advantageously, the immunomodulator, especially if ascorbic acid as is most preferred, is present in an amount of about 15 ppm to about 45 ppm.

Surprisingly, it has been found that the composition of the present invention exhibits its highest levels of antibacterial activity at a highly acidic pH, i.e., at pH of no greater than about 5, preferably a pH of no greater than about 4. In fact, the optimum pH, it has been found, is a pH of about 3.5. This runs counter to accepted belief that lysozyme is at its peak activity at a pH of 6.6 (slightly acidic) and is not effective under strongly acidic conditions. In order to maintain the pH at the desired levels, the pH of the lysozyme solution can be adjusted by a suitable acid, such as hydrochloric acid (HC1), if the composition is not brought to the preferred acidic pH by, for instance, the acidic immunomodulator.

Advantageously, to prepare the lysozyme composition of the present invention, the desired mineral component is mixed, and preferably dissolved, into a suitable solvent, most preferably a buffer solution such as a 0.1 molar (M) solution of citric acid, sodium citrate, or both, in water. The desired amounts of lysozyme and immunomodulating agent are then added to the zinc/solvent mixture and the pH adjusted with a suitable acidic agent (if necessary). The inventive lysozyme composition can be prepared in liquid form or in spray, dip or solid form, and additionally, as a mixture in fodder for poultry, cattle, other animals, and fish.

In order to reduce and/or inhibit the bacterial activity of a substrate (in other words, in order to have a bactericidal and/or bacteriostatic effect on something infected with bacteria), a bactericidal and/or bacteriostatic amount of the composition of this invention is added to the substrate. Substrates capable of being treated with the inventive composition include, but not by way of limitation, meats and meat products, such as beef, especially ground beef; mayonnaise, dairy products such as cheese, milk, yogurt, etc.; oils; fish and fish products; eggs and egg products; and other high protein products.

The bactericidal and/or bacteriostatic amount of the composition of this invention is chosen based upon the number of bacteria killed regardless of the volume or weight of substrate. This is contrary to accepted norms, where the effectiveness of a lysozyme preparation was expressed in terms of amount (e.g., milligrams) of lysozyme per unit volume (e.g., liters) or unit weight (e.g., kilograms) of substrate. In the practice of this invention, the effectiveness of the composition is measured in terms of bacteria it is capable of killing with no regard to volume or weight of substrate. For instance, if one unit of the composition is capable of killing 200,000 bacteria, then that one unit would be just as effective in 100 ml or 1 gram of substate as in several liters or kilograms of substrate.

Generally, a bactericidal and/or bacteriostatic amount of the inventive composition is considered to be about 1 to about 5 ml, more preferably about 1 to about 2 ml, with the most preferred being about 1.5 ml.

Treating the desired substrates with the lysozyme composition of the present invention can be undertaken after bacterial infection, to reduce or maintain the bacterial levels in the sample, or, most advantageously, prior to bacterial infection, to prevent or inhibit bacterial infection from occuring. In so doing, the shelf life of those foods prone to bacterial infection, such as those noted above, is extended by delaying or preventing bacterial infection and, thus, spoilage. As noted above, the lysozyme composition can also be mixed with animal fodder or feed to prevent bacterial contamination at the source (pasture, water, etc.). The inventive composition can also be used to disinfect and/or decontaminate food processing equipment and also as a preservative for solutions such as contact lens solution and pharmaceutical preparations.

Bacteria upon which this composition is known to have a positive (i.e., bactericidal and/or bacteriostatic) effect include those bacteria most often associated with food spoilage, such as salmonella typhimurium, salmonella enteriditis, Escherichia coli (E. coli), Pseudomonas aeruginosa, Staphylococcus aureus, Candida albicans, Aspergillus niger, Aspergillus fischeri, bacillus subtilis, and listeria monocytogenes, etc.

In another surprising and advantageous embodiment of the present invention, the lysozyme composition can act as both preservative preparation and curding agent on milk and milk products by "overloading" the immunomodulating agent, which is an acid. In this way, the inventive composition can be used to produce curded milk products resembling cottage cheese, white cheese and yogurt, having extended shelf life from milk products such as whole milk, skim milk, etc. These extended shelf life curded milk products also have, in many cases, different and/or improved taste and can also have other advantageous characteristics such as being vitaminated or containing aspirin, depending on the acid used.

In order to produce such improved extended shelf life curded milk products, the lysozyme composition of the present invention is prepared having the formulation described above with respect to the lysozyme and mineral components, and also the pH, but the concentration of the acidic immunomodulating agent is altered. In other words, the lysozyme present in the composition is that sufficient to provide lysozyme in an amount of about 5 to about 20 mg per liter of milk or milk product, the mineral component is present in an

5

amount of about 1.5 to about 5 mg per liter of milk or milk product and the pH is adjusted to be no greater than about 5 and most preferably about 3.5. The acidic immunomodulating agent, though, is present in this formulation in an amount of about 10 to about 120 mg per milliliter of milk or milk product.

The effects of such "overloaded" acidic component are as follows: when the amount of acidic immunomodulating agent is about 10 mg per milliliter to about 25 mg per milliliter, the color and physical appearance of milk left at room temperature, even for up to 14 days, remains the same. The taste can change, depending on the particular acid utilized. For instance, when ascorbic acid is used, the taste is more pleasant, slightly acid, with a citro-orange flavor. Untreated milk, when left at room temperature for fourteen days, changes in color, odor and taste (becoming sour), the classic characteristics of spoiled milk. When the amount of acidic immunomodulating agent is about 25 mg per milliliter to about 120 mg per milliliter, curding of the milk or milk product, in the absence of other milk curding cultures, is observed after about three to about seventy-two hours. The taste of the curded product can vary from pleasant and new, when acids such as ascorbic and pantothenic are used, to bitter when acetylsalicylic acid is used, although the bitter taste can be easily changed by use of sweeteners such as saccharin, aspartame, or sugar, syrup, honey, fruit juices, etc.

By the use of the composition of this invention, curded milk products such as vitaminated milk products (when acid vitamins such as ascorbic acid, pantothenic acid, folic acid, paraaminobenzoic acid, benzoic acid, hydrochloride acid, amino acids, etc. are used) and aspirin milk products (when acetylsalicylic acid or other salicylic acid salts are used). Such aspirin milk products are extremely advantageous due to their lessening or elimination of aspirin side effects such as stomach burns, gastric and intestinal ulcers and bleeding, especially for individuals forced to take aspirin for extended periods of time such as sufferers of chronic forms of arthritis. Of course also available and advantageous are combination vitaminated/aspirin milk products.

The following examples further illustrate and explain the present invention by the antibacterial effectiveness of compositions of the present invention.

## Example I

A lysozyme composition is prepared by: preparing a 0.1 Molar (M) citric acid buffer solution by dissolving 10.5 grams of citric acid in 500 milliliters (ml) of water and a 0.1 M sodium citrate buffer solution by dissolving 14.6 grams of sodium citrate in 500 ml of water, and combining the two solutions;

mixing 131.92 milligrams (mg) of zinc sulfate ($ZnSO_4$), which provides the equivalent of 30 mg zinc, into 20 ml of the combined buffer solution;

100 mg of hen egg white lysozyme and 300 mg of ascorbic acid are then added and the pH adjusted to 3.5 with 6 Normal (N) hydrochloric acid, to form the exemplary lysozyme composition, one cubic centimeter (cc) of which contains 5 mg of lysozyme, 15 mg of ascorbic acid, and 1.5 mg of zinc.

A comparative composition was also prepared in the same manner except that the pH was buffered at 6.6.

## Method of Assay

In order to evaluate the antibacterial capacity of the prepared compositions, serial dilutions are prepared in duplicate in 2x strength nutrient broth (commercially available from Difco Co. of Detroit, Michigan), starting from a 1:1 dilution through 1:2048.

## Preparation of Organisms

A mid-log phase culture is prepared of Salmonella typhimurium (ATCC 11331). Cells grown on nutrient agar are harvested in sterile 0.9% saline, washed twice and adjusted to achieve an inoculum concentration of about $10^7$ cells/ml as confirmed by plate counts. Ten microliters of the resulting organism suspension is used to inoculate 200 microliters of each medium, to achieve a final organism concentration of $10^5$ cells per cavity.

The inoculated micro-titer plates are incubated at 35°C for 24 hours. Plates are subsequently stored at room temperature for the balance of the test period. Subsequent visual readings of turbidity are taken at 48

EP 0 366 869 A2

hours, 72 hours and 1, 2, 3 and 4 weeks.

Minimum inhabitory concentration (MIC) reactions are scored on the basis of the development of turbidity.

In order to obtain the Minimum bacterial concentration (MBC) values, each well showing no signs of turbidity are cultured onto 5% blood agar plates to determine the presence of viable microorganisms. Growth is evaluated after 48 hours of incubation at 35.C.

The results are shown in Table 1 (for the exemplary lysozyme composition pH 3.5) and Table 1a (for the comparative composition, pH - 6.6).

## Table 1

### Cumulative Bacterial Response

| Well | Dilution | 24 hr | 48 hr | 72 hr | 1 wk | 2 wks | 3 wks |
|------|----------|-------|-------|-------|------|-------|-------|
| 1 | 1:1 | (-)x | (-)x | (-)x | (-)x | (-)x | (1)x |
| 2 | 1:2 | (-)x | (-)x | (1)x | (-)x | (1)x | (2)x |
| 3 | 1:4 | (-)x | (1)x | (1)x | (1)x | (1)x | (3)x |
| 4 | 1:8 | (-)x | (2)x | (2)x | (1)x | (2)x | (3)x |
| 5 | 1:16 | (-)x | (2) | (2) | (1)x | (2) | (4) |
| 6 | 1:32 | (1) | (4) | (4) | (1)x | (4) | (4) |

Key for Tables 1 and 1a:
x - Antibacterial Activity
(-) - No Growth
(1) - Slight Turbidity
(2) - Slight to Moderate
(3) - Moderate
(4) - Heavy Turbidity
* - Viable organisms present

7

## Table 1 (cont.)

## Cumulative Bacterial Response

| Well | Dilution | 24 hr | 48 hr | 72 hr | 1 wk | 2 wks | 3 wks |
|------|----------|-------|-------|-------|------|-------|-------|
| 7 | 1:64 | (3) | (4) | (4) | (4) | (4) | (4) |
| 8 | 1:128 | (4) | (4) | (4) | (4) | (4) | (4) |
| 9 | 1:256 | (4) | (4) | (4) | (4) | (4) | (4) |
| 10 | 1:512 | (4) | (4) | (4) | (4) | (4) | (4) |
| 11 | 1:1024 | (4) | (4) | (4) | (4) | (4) | (4) |
| 12 | 1:2048 | (4) | (4) | (4) | (4) | (4) | (4) |

The (1) and (2) turbidity markings in Wells 1, 2, 3 and 4 are due to protein precipitation/bacterial killing and are not due to bacterial growth. As noted, all tests marked with an "x" indicate bacterial killing/bactericidal effect.

## Table 1a

## Cumulative Bacterial Response

| Well | Dilution | 24 hr | 48 hr | 72 hr | 1 wk | 2 wks |
|------|----------|-------|-------|-------|------|-------|
| 1 | 1:1 | (-)* | (-)* | (-)* | (-)x | (-)x |
| 2 | 1:2 | (-)* | (1)* | (1)* | (1) | (1)x |
| 3 | 1:4 | (-)* | (2)* | (4)* | (2) | (2) |

## Table 1a (cont.)

## Cumulative Bacterial Response

| Well | Dilution | 24 hr | 48 hr | 72 hr | 1 wk | 2 wks |
|------|----------|-------|-------|-------|------|-------|
| 4 | 1:8 | (3) | (3) | (4) | (4) | (4) |
| 5 | 1:16 | (3) | (4) | (4) | (4) | (4) |
| 6 | 1:32 | (4) | (4) | (4) | (4) | (4) |
| 7 | 1:64 | (4) | (4) | (4) | (4) | (4) |
| 8 | 1:128 | (4) | (4) | (4) | (4) | (4) |
| 9 | 1:256 | (4) | (4) | (4) | (4) | (4) |
| 10 | 1:512 | (4) | (4) | (4) | (4) | (4) |
| 11 | 1:1024 | (4) | (4) | (4) | (4) | (4) |
| 12 | 1:2048 | (4) | (4) | (4) | (4) | (4) |

The results of Example I clearly show the strong antibacterial effect of the exemplary lysozyme composition. The difference between the exemplary lysozyme composition (Table 1) and the comparative composition (Table 1a) in terms of bacteria killing is striking, showing the exemplary lysozyme composition to be very effective whereas the comparative composition is not, and yet the compositions are identical except that the exemplary lysozyme composition is at pH 3.5 and the comparative composition is at pH 6.6.

## Example II

A lysozyme composition is prepared according to the method described in Example I, except that the concentration of lysozyme is varied as indicated.

## Method of Assay

In order to evaluate the antibacterial capacity of the prepared compositions, serial dilutions are prepared in duplicate in 2x strength Trypticase soy broth (commercially available from Difco Co. of Detroit, Michigan), starting from a 1:1 dilution through 1:256.

## Preparation of Organisms

9

A mid-log phase culture is prepared of Listeria monocytogenes (ATCC 7644). Cells grown on Trypticase soy agar with 5% defibrinated sheep blood are harvested in sterile 0.9% saline, washed twice and adjusted to achieve an inoculum concentration of about $10^7$ cells/ml as confirmed by plate counts. Ten microliters of the resulting organism suspension is used to inoculate 200 microliters of each medium, to achieve a final organism concentration of $10^5$ cells per cavity.

The inoculated micro-titer plates are incubated at 35°C for 24 hours. Plates are subsequently stored at room temperature for the balance of the test period. Subsequent visual readings of turbidity are taken at 24 hours, 48 hours, 72 hours and 1 week.

Minimum inhabitory concentration (MIC) reactions are scored on the basis of the development of turbidity.

In order to obtain the Minimum bacterial concentration (MBC) values, each well showing no signs of turbidity are cultured onto 5% blood agar plates to determine the presence of viable microorganisms. Growth is evaluated after 48 hours of incubation at 35°C.

The results are shown in Table 2.

## Table 2

## Cumulative Bacterial Response

| Well | Dilution | % Conc. | 24 hr | 48 hr | 72 hr | 1 week |
|------|----------|---------|-------|-------|-------|--------|
| 1 | 1:1 | 0.50 | (-)x | (-)x | (1)x | (1)x |
| 2 | 1:2 | 0.25 | (-)x | (-)x | (1)x | (1)x |
| 3 | 1:4 | 12.50 | (-)* | (-)* | (1)* | (2)* |
| 4 | 1:8 | 6.25 | (1)* | (2)* | (2)* | (2)* |
| 5 | 1:16 | 3.125 | (2)* | (2)* | (2)* | (3)* |

Key for Table 2:
x - Antibacterial Activity
(-) - No Growth
(1) - Slight Turbidity
(2) - Slight to Moderate
(3) - Moderate
(4) - Heavy Turbidity
* - Viable organisms present

10

## Table 2 (cont.)

## Cumulative Bacterial Response

| Well | Dilution | % Conc. | 24 hr | 48 hr | 72 hr | 1 week |
|------|----------|---------|-------|-------|-------|--------|
| 6 | 1:32 | 1.56 | (4) | (4) | (4) | (4) |
| 7 | 1:64 | 0.78 | (4) | (4) | (4) | (4) |
| 8 | 1:128 | 0.39 | (4) | (4) | (4) | (4) |
| 9 | 1:256 | 0.20 | (4) | (4) | (4) | (4) |

Example IIIa

Screening tests are conducted to determine the effectiveness of a lysozyme composition prepared as described in Example I against bacteria and fungi using the method of United States Pharmacopeia (USP), Chpater XIX, p. 587. The microorganisms used are Aspergillus fischeri and Bacillus subtilis. The inoculi are harvested from 24 hour slants for the bacteria and 7 days for the fungi. Initial plate counts are run to determine the level of organisms at the beginning of the test:

| | |
|---|---|
| Aspergillus fischeri, FDA M1148 | 15,000/ml |
| Bacillus subtilis, ATCC 9382 | 100,000/ml |

The concentration of lysozyme and pH, and the results, are set out in Table 3a.

Table 3a

| Aspergillus fischeri | | | | | |
|------|------|------|-------|-------|-------|
| Conc. | pH | 1 wk | 2 wks | 3 wks | 4 wks |
| 0.2 | 6.8 | <10 | <1 | <1 | <1 |
| 0.5 | 3.5 | <1 | <1 | <1 | <1 |
| Bacillus subtilis | | | | | |
| 0.2 | 6.8 | <10 | <1 | <1 | <1 |
| 0.5 | 3.5 | <1 | <1 | <1 | <1 |

Example IIIb

EP 0 366 869 A2

Screening tests are conducted to determine the effectiveness of a lysozyme composition prepared as described in Example I against bacteria and fungi using the method of United States Pharmacopeia (USP), Chpater XIX, p. 587. The inoculi are harvested from 24 hour slants for the bacteria and 7 days for the fungi. Initial plate counts are run to determine the level of organisms at the beginning of the test:

| Candida albicans, ATCC 10231 | 120,000/ml |
| Aspergillus niger, ATCC 16404 | 100,000/ml |
| Escherichia coli, ATCC 8739 | 400,000/ml |
| Pseudomonas aeruginosa, ATCC 9027 | 170,000/ml |
| Staphylococcus aureus, ATCC 6538 | 170,000/ml |

The concentration of lysozyme and pH, and the results, are set out in Table 3b.

Table 3b

| Conc. | pH | Organism | 1 wk | 2 wks | 3 wks | 4 wks |
|---|---|---|---|---|---|---|
| 0.2 | 3.5 | C. albicans | 90,000 | 45,000 | 30,000 | 15,000 |
| 0.2 | 3.5 | A. niger | 20,000 | 65,000 | 60,000 | 50,000 |
| 0.2 | 3.5 | E. coli | 55,000 | 9,000 | 2,900 | 300 |
| 0.2 | 3.5 | P. aerugin. | 75,000 | 22,000 | 11,000 | 21,000 |
| 0.2 | 3.5 | S. aureus | <1 | <1 | <1 | <1 |
| 0.5 | 3.5 | C. albicans | <1 | <1 | <1 | <1 |
| 0.5 | 3.5 | A. niger | <1 | <1 | <1 | <1 |
| 0.5 | 3.5 | E. coli | <1 | <1 | <1 | <1 |
| 0.5 | 3.5 | P. aerugin. | 20 | <1 | <1 | <1 |
| 0.5 | 3.5 | S. aureus | <1 | <1 | <1 | <1 |

Example IV

In order to evaluate the antibacterial activity of the inventive composition on fresh food, the following test samples are prepared:

Sample 1 - 50.0 grams of raw fresh ground beef ("hamburger") is weighed into a sterile container.

Sample 2 - 49.0 grams of hamburger is weighed into a sterile container and 1.0 ml of bacteria broth (comprising a 1:1 mixture of Staphylococcus aureus (ATCC 25923) broth and Salmonella enteritidis (ATCC 13076) broth, total bacterial count of at least 200,000 per ml) is added and mixed therein.

Sample 3 - 49.0 grams of hamburger is weighed into a sterile container and 0.5 ml of an exemplary lysozyme composition prepared in accordance with Example I, and 1.0 ml of bacterial broth are added and mixed therein.

Sample 4 - 49.0 grams of hamburger is weighed into a sterile container and 1.5 ml of exemplary lysozyme composition, and 1.0 ml of bacterial broth are added and mixed therein.

Sample 5 - 49.0 grams of hamburger is weighed into a sterile container and 0.5 ml of exemplary lysozyme composition added and mixed therein.

Each of the samples is stored for fourteen days at 42°F (5.6°C). An initial sample extract of 1.0 grams is taken as well as sample extracts at 24 hours, 7 days and 14 days. Each of the withdrawn 1.0 grams extracts is extracted with 100 ml of water and 0.25 to 1.0 ml plated on agar (Mannitol Salt Agar, Bismuth Sulfite Agar, MacConkey Agar and/or Brilliant Green Agar). The plates are incubated for 18 to 36 hours at 36°C and the counts recorded.

The results are interpreted by plotting all counts per cc for the five sample versus the storge times and determining the quantitative effect of the exemplary lysozyme composition. The results are set out in Tables 4a and 4b.

12

Table 4a

| Salmonella Counts | | | | | | |
|---|---|---|---|---|---|---|
| | | Days | | | | |
| Sample | Components | Initial | 1 | 7 | 14 | |
| 1 | Hamburger | 4400 | TNTC | TNTC | TNTC | |
| 2 | Hamburger Broth | 6800 | TNTC | TNTC | TNTC | |
| 3 | Hamburger Broth 0.5 ml Lysozyme Comp. | 1400 | 1300 | 600 | 600 | |
| 4 | Hamburger Broth 1.5 ml Lysozyme Comp. | 400 | 200 | 50 | N | |
| 5 | Hamburger 0.5 ml Lysozyme Comp. | 3000 | TNTC | 800 | 800 | |

Key for Tables 4a and 4b:
N - Not Detected
TNTC - Too Numerous to Count

Table 4b

| Staphylococcus Counts | | | | | | |
|---|---|---|---|---|---|---|
| | | Days | | | | |
| Sample | Components | Initial | 1 | 7 | 14 | |
| 1 | Hamburger | N | N | N | N | |
| 2 | Hamburger Broth | 6000 | TNTC | TNTC | TNTC | |
| 3 | Hamburger Broth 0.5 ml Lysozyme Comp. | 3200 | N | N | N | |
| 4 | Hamburger Broth 1.5 ml Lysozyme Comp. | N | N | N | N | |
| 5 | Hamburger 0.5 ml Lysozyme Comp. | N | N | N | N | |

The results of Example IV clearly show the bacteriostatic and bactericidal activity of the composition of the present invention in both concentrations tested.

Example V

The procedure of Example V is repeated except that instead of hamburger, mayonnaise is used as the bacterial substrate. The results are set out in Tables 5a and 5b.

Table 5a

| Salmonella Counts | | | | | |
|---|---|---|---|---|---|
| | | Days | | | |
| Sample | Components | Initial | 1 | 7 | 14 |
| 1 | Mayonnaise | N | N | N | N |
| 2 | Mayonnaise<br>Broth | 3500 | TNTC | TNTC | TNTC |
| 3 | Mayonnaise<br>Broth<br>0.5 ml Lysozyme Comp. | N | N | N | N |
| 4 | Mayonnaise<br>Broth<br>1.5 ml Lysozyme Comp. | N | N | N | N |
| 5 | Mayonnaise<br>0.5 ml Lysozyme Comp. | N | N | N | N |

Key for Tables 5a and 5b:
N - Not Detected
TNTC - Too Numerous to Count

Table 5b

| Staphylococcus Counts | | | | | |
|---|---|---|---|---|---|
| | | Days | | | |
| Sample | Components | Initial | 1 | 7 | 14 |
| 1 | Mayonnaise | N | N | N | N |
| 2 | Mayonnaise<br>Broth | 4400 | TNTC | TNTC | TNTC |
| 3 | Mayonnaise<br>Broth<br>0.5 ml Lysozyme Comp. | N | N | N | N |
| 4 | Mayonnaise<br>Broth<br>1.5 ml Lysozyme Comp. | N | N | N | N |
| 5 | Mayonnaise<br>0.5 ml Lysozyme Comp. | N | N | N | N |

The above description is for the purpose of teaching the person of ordinary skill in the art how to practice the present invention, and it is not intended to detail all of those obvious modifications and variations of it which will become apparent to the skilled worker upon reading the description. It is intended, however, that all such obvious modifications and variations be included within the scope of the present invention which is defined by the following claims.

**Claims**

1. A bactericidal, bacteriostatic and antifungal composition comprising lysozyme, characterized in that it contains additionally a mineral component and an immunomodulating agent.

2. The composition of claim wherein said lysozyme comprises hen egg white lysozyme.

3. The composition of claim 2 wherein said hen egg white lysozyme is present in an amount of 0.5 to 500 mg/ml, preferably 20 to 200 mg/ml.

4. The composition of any of claims 1 to 3 wherein said mineral component is selected from the group consisting of zinc, germanium, beryllium, chromium, tungsten, selenium, iron, copper, manganese, superoxide dismutase, magnesium, strontium and iodine/kelp, and mixtures thereof.

5. The composition of claim 4 wherein said mineral component comprises zinc and/or iodine.

6. The composition of claim 5 wherein zinc is present in an amount of 0.5 to 50 mg/ml, preferably 2 to 25 mg/ml, and/or iodine is present in an amount of 125 to 500 $\mu$g/ml.

7. The composition of any of claims 1 to 6 wherein said immunomodulating agent comprises an acid selected from the group consisting of ascorbic acid, phantothenic acid, folic acid, benzoic acid, tartaric acid, para-amino benzoic acid, linoleic acid, gammalinoleic acid, acetylsalicylic acid, caprylic acid, amino acids, fatty acids, and mixtures thereof.

8. The composition of claim 7 wherein said immunomodulating agent comprises ascorbic acid.

9. The composition of claim 8 wherein ascorbic acid is present in an amount of 6 to 300 mg/ml.

10. The composition of any of claims 1 to 9 wherein the pH is no greater than about 4.

11. The composition of claim 10 which further comprises an acidifying agent.

12. The composition of claim 7 wherein said immunomodulating agent comprises at least one saturated or unsaturated fatty acid.

13. The composition of claim 12 wherein said at least one fatty acid is present in an amount of 50 to 500 mg/ml.

14. The composition of claims 12 or 13, wherein the pH is 3.5 to 12.

15. A bactericidal, bacteriostatic and antifungal composition capable of exerting its bactericidal, bacteriostatic and antifungal effect on a number of bacteria and fungi regardless of the volume or weight of bacteriological substrate, according to any of claims 1 to 14, comprising lysozyme, a mineral component, an immunomodulating agent and further wherein the pH of the composition is no greater than about 5.

16. A method for reducing or inhibiting the bacteriological or fungal activity in a substrate comprising adding a bacteriostatic, bactericidal or antifungal amount of a composition according to any of claims 1 to 15 to the said substrate.

17. A method for the extension of the shelf life of fresh foods comprising adding a shelf-extending amount of a composition according to anyone of claims 1 to 15 to the said foods.

18. The method of claim 17 wherein the fresh food whose shelf life is extended is selected from the group consisting of ground beef, mayonnaise, egg nog, low cholesterol foods using modified egg yolk, hot dogs, oils, cheese, milk, yogurt, soft drinks and fish products.

19. The method of claim 17 comprising adding to milk or a milk product a composition comprising 20 milligrams of lysozyme per liter of milk or milk product, 1.5 to 5 milligrams of a mineral component per liter of milk or milk product, 10 to 120 milligrams of an immunomodulating agent per milliliter of milk or milk product and further wherein the pH of the composition is no greater than about 5.

20. The method of claim 18 wherein said immunomodulating agent comprises acetylsalicylic acid.

21. A method for inducing the curding of milk and milk products while extending the shelf life thereof, comprising adding to milk or a milk product the composition of claim 19 wherein said immunomodulating agent is present in an amount of 25 to 120 mg/ml of milk or milk product.

22. Use of the composition of any of claims 1 to 15 for the formation of a bacteriostatic, bactericidal and/or antifungal substrate in solid or liquid form.

23. The use of claim 22, which comprises applying 1 ml of the said composition to 1 kg or 1 l of the said substrate.

24. The use of claim 22 or 23, wherein the said substrate is a food product.

25. The use of claim 22 or 23, wherein the said substrate is a mouthwashing composition, a dental cleaning agent, a contact lens cleaining solution, a cosmetic composition, a composition for feminine hygiene, a diaper, a medical or nonmedical desinfectant, a composition for treatment of bedsore, or an animal feed.

26. A substrate in liquid or solid form, treated with the composition of any of claims 1 to 15 in an amount of about 1 ml per l or kg.